# EUROPÄISCHE PATENTSCHRIFT

(11) **EP 2 736 327 B1**
(45) Veröffentlichungstag und Bekanntmachung des Hinweises auf die Patenterteilung: **21.02.2018**
(21) Anmeldenummer: 12738129.1
(22) Anmeldetag: 23.07.2012
(51) Int. Cl.: A01N 25/22, C07C 67/31, C07C 69/708, A01N 25/30, A01N 37/36

(54) **VERETHERTE LAKTATESTER, VERFAHREN ZU IHRER HERSTELLUNG UND IHRE VERWENDUNG ZUR VERBESSERUNG DER WIRKUNG VON PFLANZENSCHUTZMITTELN**
ETHERIFIED LACTATE ESTERS, METHOD FOR PRODUCING SAME AND USE OF SAME TO IMPROVE THE EFFECT OF PLANT PROTECTING AGENTS
ESTER DE LACTATE ETHÉRIFIÉ, SON PROCÉDÉ DE FABRICATION ET SON UTILISATION POUR L'AMÉLIORATION DE L'EFFET DE PRODUITS PHYTOSANITAIRES

(30) Priorität: 26.07.2011 EP 11175341; 26.07.2011 US 201161511601 P
(43) Veröffentlichungstag der Anmeldung: 04.06.2014
(73) Patentinhaber: Clariant International Ltd, 4132 Muttenz (CH)
(72) Erfinder: BAUR, Peter, 86938 Schondorf (DE); LORENZ, Klaus, 41539 Dormagen (DE); HOFMANN, Jörg, 47800 Krefeld (DE)
(74) Vertreter: Paczkowski, Marcus
(86) Internationale Anmeldenummer: PCT/EP2012/064421
(87) Internationale Veröffentlichungsnummer: WO 2013/014126

(56) Entgegenhaltungen:
- EP-A1- 1 702 941
- WO-A2-2007/028538
- DE-A1-102007 018 983
- JP-A- 6 122 655
- DATABASE CAPLUS [Online] CHEMICAL ABSTRACTS SERVICE, COLUMBUS, OHIO, US; SAITO, YUTAKA ET AL: "Preparation of 1a-(acyloxymethoxycarbonyl)mitomycin C as prodrugs", XP002666948, gefunden im STN Database accession no. 1989:614326 & JP 1 113391 A (KYOWA HAKKO KOGYO CO., LTD., JAPAN) 2. Mai 1989 (1989-05-02)

## Beschreibung

Die Erfindung betrifft neue veretherte Laktatester, Verfahren zu ihrer Herstellung und ihre Verwendung zur Verbesserung der Wirkung von agrochemischen Wirkstoffen in und auf Pflanzen.

WO-A-1991/14366 beschreibt Laktat und Laktat-Derivate als Wirkstoffe, die das Wachstum von Weintrauben regulieren. Die Substanzen werden hier in einer festgelegten Dosierung ohne weitere Zusätze direkt in Wasser angesetzt und als Blattspritzlösung in einem frühen Wachstumsstadium der Weintrauben verwendet. Der Einsatz von Laktatderivaten zur Verbesserung der Wirkung von Pflanzenschutzmitteln auf der Ebene der Pflanzen wird in diesem Dokument jedoch weder offenbart noch nahelegt.

In WO-A-2000/18227 werden Alkyllaktate in hohen Konzentrationen als Suspensionsmittel für nicht lösliche agrochemische Wirkstoffe in nicht-wässrigen Suspensionskonzentraten beschrieben. Der Einsatz von Alkyllaktaten zur Verbesserung der Wirkung auf der Ebene der Pflanzen wird in diesem Dokument weder offenbart noch nahe gelegt. WO-A-2000/18227 offenbart auch keine veretherten Laktatester.

In WO-A-2003/075657 werden Laktatester in hohen Konzentrationen als Kristallisations-inhibitoren und Lösungsmittel für nicht lösliche agrochemische Wirkstoffe, insbesondere Azol-Fungizide beschrieben. Der Einsatz von Laktatestern zur Verbesserung der Wirkung auf der Ebene der Pflanzen wird auch in diesem Dokument weder offenbart noch nahegelegt. WO-A-2003/075657 offenbart auch keine veretherten Laktatester.

WO-A-1996/22020 beschreibt die Verwendung von aliphatischen Estern als Penetrationsförderer. WO-A-1996/22020 offenbart aber keine veretherten Laktatester.

In WO-A-2007/028538 werden Laktatester mit freier Hydroxyfunktion zur Verbesserung der Wirksamkeit von Pflanzenschutzmitteln beschrieben. WO-A-2007/028538 offenbart aber keine veretherten Laktatester.

EP-B-1 702 941 beschreibt ein Verfahren zur Herstellung von Poly(etherester)polyolen bei dem in einem ersten Schritt ein oder mehrere Hydroxygruppen enthaltende Mono- oder Polycarbonsäureester mit Alkylenoxiden unter Ringöffnung in Gegenwart von DMC-Katalysatoren (Doppelmetallcyanidkomplex-Katalyse) zu den entsprechenden Mono- oder Polycarbonsäureestern mit ein oder mehreren angebundenen Polyetherketten umgesetzt werden, wobei die Alkylenoxidaddition gegebenfalls in Gegenwart eines Di- oder Polyols durchgeführt wird und in einem zweiten Schritt anschließend die in Schritt a) hergestellten Produkte zu OH-funktionellen Poly(etherester)n umgeestert werden ([0015]). Als Ausgangskomponenten für die Herstellung der Mono- oder Polycarbonsäureester mit angebundenen Polyetherketten eignen sich unter anderem die Ester der Milchsäure ([0016]). Konkrete Ester der Milchsäure werden nicht genannt. Die erfindungsgemäßen veretherten Laktatester werden ebenfalls nicht offenbart. Die Details des Verfahrens werden in ([0017]) - ([0029]) beschrieben. Die so hergestellten Poly(etherester)polyolen werden nach EP-B-1 702 941 als Ausgangsstoffe für die Herstellung von Polyurethanwerkstoffen genutzt. JP 6122655A offenbart die Verwendung von Laktatestern als Detergenzien.

Nicht beschrieben wurde bisher eine Anwendung von an der Hydroxygruppe veretherten Laktatestern (Laktatester-Alkoxylate) als Verbesserer der Wirkung von agrochemischen Wirkstoffen in Pflanzenschutzmitteln. Dabei wird unter Pflanzenschutzmitteln die Anwendungsform der agrochemischen Wirkstoffe verstanden, z.B. die Spritzbrühe.

Es wurde nun überraschend gefunden, dass die Wirkung von Pflanzenschutzmitteln auf der Ebene der Pflanzen signifikant durch bestimmte, veretherte Laktatester verbessert wird. So fördern die erfindungsgemäßen veretherten Laktatester als Netzmittel sowohl den Verbleib der Spritzbrühe der die agrochemischen Wirkstoffe enthaltenden Pflanzenschutzmittel auf der Pflanze, vor allem den Blättern (verbesserte Retention), als auch das Eindringen der in den Pflanzenschutzmitteln enthaltenen agrochemischen Wirkstoffe in die Pflanze (verbesserte Penetration). Diese Verbesserung der Eigenschaften wird bereits bei Konzentrationen von veretherten Laktatestern erreicht, die denen von typischen Netzmitteln oder Penetrationsförderern entsprechen.

Gegenstand der Erfindung sind veretherte Laktatester der Fomel (I) 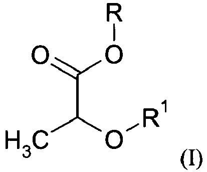 worin
- R: für 2-Ethyl-Hexyl oder Lauryl steht,
- R¹: für einen alkoxylierten Alkylrest der Formel -(-AO)ₘ-R' steht, wobei
- AO: für einen Ethylenoxid-Rest, einen Propylenoxid-Rest, einen Butylenoxid-Rest oder für Gemische aus Ethylenoxid- und Propylenoxid-Resten steht und
- m: für Zahlen von 2 bis 15 steht,
- R': für Wasserstoff oder für einen verzweigten oder unverzweigten, gesättigten, teilweise gesättigten oder ungesättigten C₁-C₁₀Alkylrest steht.

Dabei werden der Ethylenoxid-Rest, der Propylenoxid-Rest und der Butylenoxid-Rest im Folgenden auch einfach als EO, PO bzw. BO bezeichnet.

Das wesentliche strukturelle chemische Element, das die erfindungsgemäßen veretherten Laktatestern von den Laktatestern aus dem Stand der Technik unterscheidet ist somit, dass die erfindungsgemäßen veretherten Laktatestern alkoxyliert sind.

Bevorzugt sind die folgenden Verbindungen, bei denen R für 2-Ethyl-Hexyl oder Lauryl **(**C₁₂) und R¹ für einen alkoxylierten Alkylrest der Formel -(-AO)ₘ-R', wobei R' für Wasserstoff oder Methyl steht, wobei m für Zahlen von 2 bis 15 steht und AO die oben angegebene Bedeutung hat.

Ganz besonders bevorzugt sind die folgenden Verbindungen:
Verethertes Lauryllaktat, d.h. R steht für Lauryl (C₁₂-Alkyl), R1 steht für -(-AO)ₘ-R', wobei R' für Wassserstoff steht und -(-AO)ₘ aus der Gruppe bestehend aus den folgenden Alkyoxylat-Resten ausgewählt ist: -(EO)₅-(PO)₂, -(EO)₅-(PO)₅, -(EO)₈-(PO)₂, -(EO)₈-(PO)₅.

Verethertes 2-Ethylhexyllaktat, d.h. R steht für 2-Ethylhexyl (-CH₂-CH(C₂H₅)-(CH₂)₃-CH₃), R1 steht für -(-AO)ₘ-R', wobei R' für Wasserstoff steht und -(-AO)ₘ aus der Gruppe bestehend aus den folgenden Alkyoxylat-Resten ausgewählt ist: -(EO)₂-(PO)₂, -(EO)₂-(PO)₅, -(EO)₂-(PO)₁₀, -(EO)₂, -(EO)₅, -(EO)₁₀,-(EO)₁₅.

Die hier beschriebenen erfindungsgemäßen veretherten Laktatester umfassen dabei sämtliche Enantiomeren. Bevorzugt liegen die erfindungsgemäßen veretherten Laktatester in der (S)-Form vor, die (R)-Form ist aber ebenfalls gut einsetzbar.

Die erfindungsgemäßen veretherten Laktatester können nach dem in EP-B-1 702 941 beschriebenen Verfahren hergestellt werden. Dazu werden die Laktatester der Formel (II), in der R die oben angegebenen Bedeutung hat und in der R2 für R' steht, wobei R' die oben angegebene Bedeutung hat 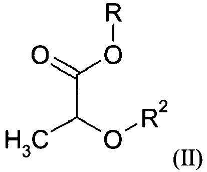 mit Alkylenoxiden (EO, PO, BO oder Gemischen daraus) in Gegenwart von DMC-Katalysatoren (Doppelmetallcyanidkomplex-Katalyse) hergestellt. Die Verfahrensbedingungen, der Verfahrensablauf und der Katalysator sind aus EP-B-1 702 941 prinzipiell bekannt. Diesbezüglich wird auf EP-B-1 702 941, insbesondere ([0015]) - ([0029]) Bezug genommen.

Die als Vorstufe eingesetzten Laktatester der Formel (II) sind kommerziell erhältlich. Das Verfahren kann dabei wie folgt durchgeführt werden:
Für das erfindungsgemäße Verfahren geeignete DMC-Katalysatoren sind im Prinzip aus dem Stand der Technik bekannt (siehe z.B. US-A 3 404 109, US-A 3829505, US-A 3941849 und US-A 5158922). DMC-Katalysatoren, die z.B. in US-A 5470813, EP-A 700949, EP-A 743093, EP-A 761708, WO 97/40086, WO 98/16310, WO 00/47649 und WO 01/80994 beschrieben sind, besitzen eine sehr hohe Aktivität in der Polymerisation von Alkylenoxiden und ermöglichen die Herstellung von Polyethern unter optimalen Bedingungen bei sehr geringen Katalysatorkonzentrationen (100 ppm oder weniger), so dass eine Abtrennung des Katalysators aus dem fertigen Produkt im Allgemeinen nicht mehr erforderlich ist. Ein typisches Beispiel sind die in EP-A 700949 beschriebenen hochaktiven DMC-Katalysatoren, die neben einer Doppelmetallcyanid-Verbindung (z.B. Zinkhexacyanocobaltat(III)) und einem organischen Komplexliganden (z.B. tert.-Butanol) noch einen Polyether mit einem zahlenmittlerem Molekulargewicht größer als 500 g/mol enthalten.

Die als Starterkomponenten erfindungsgemäß eingesetzten Laktatester der Formel (II) können im Reaktor vorgelegt werden oder dem Reaktor während der Reaktion gemeinsam mit den Alkylenoxiden kontinuierlich zugeführt werden. Bei letzterer Verfahrensweise wird im Reaktor üblicherweise eine kleine Menge eines Additionsproduktes aus Laktatester der Formel (II) und Alkylenoxid vorgelegt, dies kann auch das herzustellende Produkt sein. Es ist ebenso möglich, Reaktionsprodukt kontinuierlich dem Reaktor zu entnehmen, hierbei muss neben Alkylenoxid und der Starterkomponente auch der DMC-Katalysator kontinuierlich zudosiert werden. Die Verfahrensvarianten zur Herstellung von Alkylenoxidadditionsprodukten unter DMC-Katalyse mit kontinuierlicher Dosierung der Starterkomponenten sind z.B. beschrieben in WO 97/29146 und WO 98/03571.

Die DMC-katalysierte Reaktion der Laktatester der Formel (II) mit den Alkylenoxiden erfolgt im Allgemeinen bei Temperaturen von 20 bis 200°C, bevorzugt von 40 bis 180°C, besonders bevorzugt bei Temperaturen von 50 bis 150°C. Die Reaktion kann bei Gesamtdrücken von 0,0001 bis 20 bar (absolut) durchgeführt werden. Die Polyaddition kann in Substanz oder einem inerten organischen Lösungsmittel wie Toluol und/oder THF durchgeführt werden. Die Menge an Lösungsmittel beträgt üblicherweise 10 bis 30 Gew.-%, bezogen auf die Menge des herzustellenden veretherten Laktatesters.

Die Katalysatorkonzentration wird so gewählt, dass unter den gegebenen Reaktionsbedingungen eine gute Beherrschung der Polyadditionsreaktion möglich ist. Die Katalysatorkonzentration beträgt im Allgemeinen 0,0005 Gew.-% bis 1 Gew.-%, bevorzugt 0,001 Gew.-% bis 0,1 Gew.-%, besonders bevorzugt 0,001 bis 0,03 Gew.-%, bezogen auf die Menge des herzustellenden veretherten Laktatesters. Den als Starterkomponenten erfindungsgemäß eingesetzten Laktatestern der Formel (II) können geringe Mengen (1 - 500 ppm, bezogen auf die Startermenge) organischer oder anorganischer Säuren, wie in WO 99/14258 beschrieben, zugesetzt werden.

Den auf diese Weise hergestellten veretherten Laktatestern können ggf. Alterungsschutzmittel wie z. B. Antioxidantien zugesetzt werden.

Das Verfahren zur Herstellung der erfindungsgemäßen veretherten Laktatester ist ebenfalls Gegenstand des Verfahrens.

Gegenstand der Erfindung ist auch die Verwendung der erfindungsgemäßen veretherten Laktatester der Formel (I) 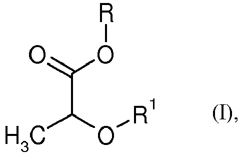 R für unverzweigtes oder verzweigtes, gesättigtes oder ungesättigtes C1-C20-Alkyl steht, und R¹ für einen alkoxylierten Alkylrest der Formel -(-AO)m-R' steht, wobei AO für einen Ethylenoxid-Rest, einen Propylenoxid-Rest, einen Butylenoxid-Rest oder für Gemische aus Ethylenoxidund Propylenoxid-Resten oder für Gemische aus Ethylenoxidund Butylenoxid-Resten steht und m für Zahlen von 2 bis 30 steht, R' für Wasserstoff oder für einen verzweigten oder unverzweigten, gesättigten, teilweise gesättigten oder ungesättigten C1-C20 Alkylrest steht, zur Verbesserung der Wirkung von agrochemischen Wirkstoffen in und auf Pflanzen. Besonders bevorzugt ist dabei die Verbesserung der Penetration von agrochemischen Wirkstoffen in Pflanzen und die Verbesserung der Retention von agrochemischen Wirkstoffen an Pflanzen, insbesondere an Blättern.

Gegenstand der Erfindung ist auch die Verwendung der erfindungsgemäßen veretherten Laktatester als Tensid, als Netz- und Haftmittel sowie als Emulgator.

Überraschenderweise wurde gefunden, dass viele der erfindungsgemäßem veretherten Laktatester ein ausgezeichnetes Schäumungsverhalten zeigen, das heißt insbesondere in wässrigen Systemen nur wenig schäumen. Somit ist Gegenstand der Erfindung auch die Verwendung der erfindungsgemäßen veretherten Laktatester zur Vermeidung oder Reduktion der Schaumbildung in agrochemischen Formulierungen. Denn bei Verwendung anderer Penetrationsförderer kommt es oft zu verstärkter Schaumbildung und damit der Notwendigkeit, zusätzlich Entschäumer einzusetzen.

Die Verbindungen der Formel (I) werden einzeln oder in Form von Gemischen eingesetzt. Wird in der Beschreibung oder den Ansprüchen von veretherten Laktatestern gesprochen, so sind ausdrücklich einzelne erfindungsgemäße Verbindungen oder Mischungen von mehreren erfindungsgemäßen Verbindungen gemeint.

Die erfindungsgemäß verwendeten veretherten Laktatester können gegebenenfalls als Mischungen verschiedener möglicher isomerer Formen, insbesondere von Stereoisomeren, wie z.B. E- und Z-, threo- und erythro-, sowie optischen Isomeren, vorliegen. Bevorzugt werden L-Laktat-Derivate der Formel (I) eingesetzt.

Die Menge an einer oder mehreren Verbindungen der Formel (I) bei der erfindungsgemäßen Verwendung in Pflanzenschutzmitteln kann nach Wirkstoff und Formulierungstyp in weiten Grenzen variieren. Die Verbindungen der Formel (I) können in allen üblichen agrochemischen Formulierungen eingesetzt werden, bevorzugt in flüssigen. Gegenstand der vorliegenden Erfindung ist auch die Verwendung der veretherten Laktatester der Formel (I) zur Verbesserung der Wirkung auf Ebene der Pflanze als Tank-Mix-Additiv, d. h. dass die veretherten Laktatester erst direkt vor dem Ausbringen einer aus einer konzentrierten Formulierung hergestellten Spritzbrühe zugesetzt werden. Prinzipiell können die Verbindungen aber auch in feste Formulierungen eingebracht werden.

Die erfindungsgemäße Verwendung der veretherten Laktatester der Formel (I) erfolgt beispielsweise in anwendungsfertigen Pflanzenschutzmitteln (Spritzbrühen), in denen der Gehalt an einem oder mehreren veretherten Laktatestern der Formel (I)
- 0,01 bis 3 Gew.-%,
- besonders bevorzugt 0,01 bis 1 Gew.-%,
- ganz besonders bevorzugt 0,02 bis 0,5 Gew.-%,
- insbesondere bevorzugt 0,03 bis 0,3 Gew.-%
beträgt.

Enthält ein Pflanzenschutzmittel mehrere veretherten Laktatester, so ist die Mengenangabe als Gesamtgehalt aller veretherten Laktatester zu verstehen.

Die oben aufgeführten allgemeinen oder in Vorzugsbereichen aufgeführten Restedefinitionen, Wertebereiche bzw. Erläuterungen können auch untereinander, also zwischen den jeweiligen Bereichen und Vorzugsbereichen beliebig kombiniert werden.

Da der Wirkmechanismus der veretherten Laktatester als Penetrationsförderer grundsätzlich unabhängig von der Art des eingesetzten agrochemischen Wirkstoffs ist, kommt ihre Verwendung in Pflanzenschutzmitteln enthaltend mindestens einen Wirkstoff, dessen biologische Wirksamkeit durch erhöhtes Eindringen in eine Kultur- oder Schadpflanze erhöht werden kann, in Frage.

Da auch der Wirkmechanismus der veretherten Laktatester als Retentionsförderer grundsätzlich unabhängig von der Art des eingesetzten agrochemischen Wirkstoffs ist, kommt ihre Verwendung in Pflanzenschutzmitteln enthaltend mindestens einen Wirkstoff, dessen biologische Wirksamkeit durch verbesserte Retention auf der Kultur- oder Schadpflanze erhöht werden kann, in Frage.

Vorzugsweise genannt seien Fungizide, Bakterizide, Insektizide, Akarizide, Nematizide, Herbizide, Pflanzenwuchsregulatoren, Pflanzennährstoffe und Repellents.

Als Beispiele für Fungizide seien genannt:
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid, 5,8-Difluor-N-[2-(2-fluor-4-{[4-(trifluormethyl)pyridin-2-yl]oxy}phenyl)ethyl]quinazolin-4-amin, N-[9-(Dichlormethylen)-1,2,3,4-tetrahydro-1,4-methanonaphthalen-5-yl]-3-(difluormethyl)-1-methyl-1H-pyrazol-4-carboxamid, N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Coumethoxystrobin, Coumoxystrobin, Dimoxystrobin, Enestroburin, Famoxadon, Fenamidon, Fenoxystrobin, Fluoxastrobin, Kresoxim-Methyl, Metominostrobin, Orysastrobin, Picoxystrobin, Pyraclostrobin, Pyrametostrobin, Pyraoxystrobin, Pyribencarb, Triclopyricarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim, Pyrimethanil und 3-(5-Fluor-3,3,4,4-tetramethyl-3,4-dihydroisoquinolin-1-yl)quinolin.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon, Tricyclazol und 2,2,2-Trifluorethyl {3-methyl-1-[(4-methylbenzoyl)amino]butan-2-yl}carbamat.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl und Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Pyriofenon (Chlazafenon), Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrimorph, (2E)-3-(4-Tert-butylphenyl)-3-(2-chlorpyridin-4-yl)-1-(morpholin-4-yl)prop-2-en-1-on, (2Z)-3-(4-Tert-butylphenyl)-3-(2-chlorpyridin-4-yl)-1-(morpholin-4-yl)prop-2-en-1-on, Pyrrolnitrin, Tebufloquin, Tecloftalam, Tolnifanid, Triazoxid, Trichlamid, Zarilamid, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2,6-Dimethyl-1H,5H-[1,4]dithiino[2,3-c:5,6-c']dipyrrol-1,3,5,7(2H,6H)-tetron, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3-(4,4,5-Trifluor-3,3-dimethyl-3,4-dihydroisoquinolin-1-yl)quinolin, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Fluor-2-[(4-fluorbenzyl)oxy]pyrimidin-4-amin, 5-Fluor-2-[(4-methylbenzyl)oxy]pyrimidin-4-amin, 5-Methyl-6-octyl[1,2,4]triazolo[1,5-a]pyrimidin-7-amin, Ethyl-(2Z)-3-amino-2-cyan-3-phenylprop-2-enoat, N'-(4-{[3-(4-Chlorbenzyl)-1,2,4-thiadiazol-5-yl]oxy}-2,5-dimethylphenyl)-N-ethyl-N-methylimidoformamid, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)-imino][6-(difluormethoxy)-2,3-difluorphenyl]methyl}-2-phenylacetamid, N'-{4-[(3-Tert-butyl-4-cyano-1,2-thiazol-5-yl)oxy]-2-chlor-5-methylphenyl}-N-ethyl-N-methylimidoformamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}-piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol, Chinolin-8-olsulfat(2:1) und Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-flor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon, N-[2-(4-{[3-(4-Chlorphenyl)prop-2-in-1-yl]oxy}-3-methoxyphenyl)ethyl]-N2-(methylsulfonyl)valinamid, 4-Oxo-4-[(2-phenylethyl)amino]butansäure und But-3-yn-1-yl {6-[({[(Z)-(1-methyl-1H-tetrazol-5-yl)(phenyl)methylen]amino}oxy)methyl]pyridin-2-yl}carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Als Beispiele für Bakterizide seien genannt:
Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere KupferZubereitungen.

Als Beispiele für Insektizide, Akarizide und Nematizide seien genannt:
Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
   (1) Acetylcholinesterase (AChE) Inhibitoren, wie beispielsweise
      Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
      Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemetonmethyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
   (2) GABA-gesteuerte Chlorid-Kanal-Antagonisten, wie beispielsweise
      Cyclodien-organochlorine, z.B. Chlordane und Endosulfan; oder
      Phenylpyrazole (Fiprole), z.B. Ethiprole und Fipronil.
   (3) Natrium-Kanal-Modulatoren / Spannungsabhängige Natrium-Kanal-Blocker, wie beispielsweise
      Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
      DDT; oder Methoxychlor.
   (4) Nikotinerge Acetylcholin-Rezeptor (nAChR) Agonisten, wie beispielsweise
      Neonikotinoide, z.B. Acetamiprid, Clothianidin, Dinotefuran, Imidacloprid, Nitenpyram, Thiacloprid und Thiamethoxam; oder
      Nikotin.
   (5) Nikotinerge Acetylcholin-Rezeptor (nAChR) allosterische Aktivatoren, wie beispielsweise
      Spinosine, z.B. Spinetoram und Spinosad.
   (6) Chlorid-Kanal-Aktivatoren, wie beispielsweise
      Avermectine/Milbemycine, z.B. Abamectin, Emamectin-benzoat, Lepimectin und Milbemectin.
   (7) Juvenilhormon-Imitatoren, wie beispielsweise
      Juvenilhormon-Analoge, z.B. Hydroprene, Kinoprene und Methoprene; oder
      Fenoxycarb; oder Pyriproxyfen.
   (8) Wirkstoffe mit unbekannten oder nicht spezifischen Wirkmechanismen, wie beispielsweise
      Alkylhalide, z.B. Methylbromid und andere Alkylhalide; oder
      Chloropicrin; oder Sulfurylfluorid; oder Borax; oder Brechweinstein.
   (9) Selektive Fraßhemmer, z.B. Pymetrozine; oder Flonicamid.
   (10) Milbenwachstumsinhibitoren, z.B. Clofentezine, Hexythiazox und Diflovidazin; oder
      Etoxazole.
   (11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
   (12) Inhibitoren der oxidativen Phosphorylierung, ATP-Disruptoren, wie beispielsweise Diafenthiuron; oder
      Organozinnverbindungen, z.B. Azocyclotin, Cyhexatin und Fenbutatin-oxid; oder
      Propargite; oder Tetradifon.
   (13) Entkoppler der oxidativen Phoshorylierung durch Unterbrechung des H-Protongradienten, wie beispielsweise Chlorfenapyr, DNOC und Sulfluramid.
   (14) Nikotinerge Acetylcholin-Rezeptor-Antagonisten, wie beispielsweise Bensultap, Cartap-hydrochlorid, Thiocyclam und Thiosultap-Natrium.
   (15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
   (16) Inhibitoren der Chitinbiosynthese, Typ 1, wie beispielsweise Buprofezin.
   (17) Häutungsstörende Wirkstoffe, Dipteran, wie beispielsweise Cyromazine.
   (18) Ecdyson-Rezeptor Agonisten, wie beispielsweise Chromafenozide, Halofenozide, Methoxyfenozide und Tebufenozide.
   (19) Oktopaminerge Agonisten, wie beispielsweise Amitraz.
   (20) Komplex-III-Elektronentransportinhibitoren, wie beispielsweise Hydramethylnon; oder Acequinocyl; oder Fluacrypyrim.
   (21) Komplex-I-Elektronentransportinhibitoren, beispielsweise
      METI-Akarizide, z.B. Fenazaquin, Fenpyroximate, Pyrimidifen, Pyridaben, Tebufenpyrad und Tolfenpyrad; oder
      Rotenone (Derris).
   (22) Spannungsabhängige Natriumkanal-Blocker, z.B. Indoxacarb; oder Metaflumizone.
   (23) Inhibitoren der Acetyl-CoA-Carboxylase, wie beispielsweise
      Tetron- und Tetramsäurederivate, z.B. Spirodiclofen, Spiromesifen und Spirotetramat.
   (24) Komplex-IV-Elektronentransportinhibitoren, wie beispielsweise
      Phosphine, z.B. Aluminiumphosphid, Calciumphosphid, Phosphin und Zinkphosphid; oder
      Cyanid.
   (25) Komplex-II-Elektronentransportinhibitoren, wie beispielsweise Cyenopyrafen.
   (28) Ryanodinrezeptor-Effektoren, wie beispielsweise
      Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Pyridalyl, Pyrifluquinazon und Iodmethan; desweiteren Präparate auf Basis von Bacillus firmus (I-1582, BioNeem, Votivo) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), 4-{[(6-Chlorpyrid-3-yl)methyl](2,2-difluorethyl)amino}furan-2(5H)-on (bekannt aus WO2007/115644), 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0539588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Sulfoxaflor (ebenfalls bekannt aus WO2007/149134) und seine Diastereomere [(R)-Methyl(oxido){(1R)-1-[6-(trifluormethyl)pyridin-3-yl]ethyl}-λ⁴-sulfanyliden]-cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), 2-Ethyl-7-methoxy-3-methyl-6-[(2,2,3,3-tetrafluor-2,3-dihydro-1,4-benzodioxin-6-yl)oxy]chinolin-4-yl-methylcarbonat (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (bekannt aus JP2008/110953), PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl]-(2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502) und (1E)-N-[(6-Chlorpyridin-3-yl)methyl]-N'-cyan-N-(2,2-difluorethyl)ethanimidamid (bekannt aus WO2008/009360).

Als Beispiele für Herbizide seien genannt:
Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolaktat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2006 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
   Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminocyclopyrachlor-kalium, Aminocyclopyrachlor-methyl, Aminopyralid, Amitrole, Ammoniumsulfamat, Ancymidol, Anilofos, Asulam, Atrazine, Aviglycin, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Benzyladenin, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbaryl, Carbetamide, Carfentrazone, Carfentrazone-ethyl, Carvone, Chlorcholinchlorid, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, 4-Chlorophenoxyacetic acid, Chlorophthalim, Chlorpropham, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cloxyfonac, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, Cytokinine, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Diaminozid, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyr-natrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Diisopropylnaphthalene, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethylnaphthylacetat, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl) pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methylsodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosateisopropylammonium, H-9201, d. h. O-(2,4-Dimethyl-6-nitrophenyl)-O-ethyl-isopropylphosphor amidothioat, Halosafen, Halosulfuron, Halosulfuron-methyl, Haloxyfop, Haloxyfop-P, Haloxyfopethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl-(2,4-dichlorphenoxy)acetat, Imazamethabenz, Imazamethabenzmethyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-Indol-3-ylbuttersäure (IBA), Iodosulfuron, Iodosulfuron-methyl-natrium, Iofensulfuron, Iofensulfuron-natrium, Ioxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamidedihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, 1-Naphtylacetic Acid (NAA), Naphthylacetamid (NAAm), 2-Naphtoxyacetic Acid, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitroguaiacolate, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenopbutyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SW-065, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamone, Triallate, Triasulfuron, Triaziflam, Triazofenamide, Tribenuron, Tribenuron-methyl, Tribufos, Trichloressigsäure (TCA), Triclopyr, Tridiphane, Trietazine, Trifloxysulfuron, Trifloxysulfuron-natrium, Trifluralin, Triflusulfuron, Triflusulfuronmethyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

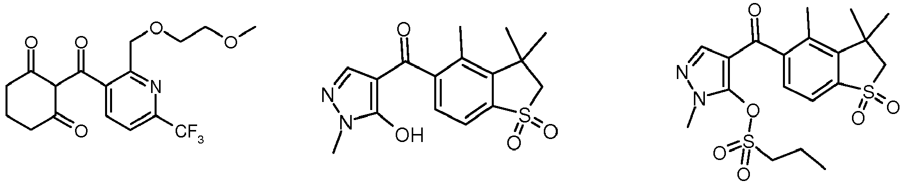

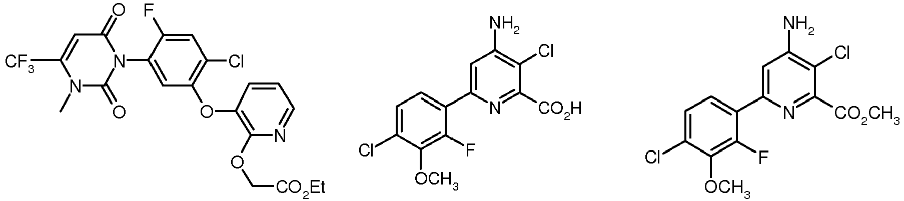

Als Beispiele für Pflanzenwuchsregulatoren seien ferner genannt natürliche Pflanzenhormone wie Abszissinsäure, Jasmonsäure, Salicylsäure bzw. deren Ester, Kinetin und Brassinosteroide

Als Beispiele für Pflanzennährstoffe seien übliche anorganische oder organische Dünger zur Versorgung von Pflanzen mit Makro- und/oder Mikronährstoffen genannt.

Als Beispiele für Repellents seien Diethyltolylamid, Ethylhexandiol und Butopyronoxyl genannt.

Bevorzugte agrochemische Wirkstoffe sind Butenolide, Neonicotinoide, Triazole und Strobilurine, insbesondere Flupyradifurone, Imidacloprid, Thiacloprid, Cyproconazole, Epoxiconazole, Metconazole, Prothioconazole, Tebuconazole sowie Azoxystrobin, Fluoxastrobin, Kresoxim-methyl, Metominostrobin, Picoxystrobin, Pyraclostrobin und Trifloxystrobin. Ebenfalls bevorzugt sind alle systemischen blattapplizierten bzw. Nachauflauf-Herbizide und Safener, Insbesondere Amidosulfuron, Bromoxynil, Cypsosulfamide, 2,4-D, Glufosinat, Glyphosat, Iodosulfuron-methyl, Isoxadifen-ethyl, Mefenpyr, Mesosulfuron, Mesotrione, Metamitron, Phenmedipham, Sulcotrione, Tembotrione und Thiencarbazone-methyl.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Der Gehalt an den einzelnen Komponenten kann in den erfindungsgemäßen Formulierungen innerhalb eines größeren Bereichs variiert werden.

Die Herstellung der erfindungsgemäßen Formulierungen erfolgt z.B. in der Weise, dass man die Komponenten in den jeweils gewünschten Verhältnissen miteinander vermischt. Handelt es sich bei dem agrochemischen Wirkstoff um eine Festsubstanz, so setzt man diesen im allgemeinen entweder in fein gemahlener Form oder in Form einer Lösung oder Suspension in einem organischen Solvens oder Wasser ein. Ist der agrochemische Wirkstoff flüssig, so erübrigt sich häufig die Verwendung eines organischen Lösungsmittels. Es ist außerdem möglich, einen festen agrochemischen Wirkstoff in Form einer Schmelze einzusetzen.

Die Temperaturen können bei der Durchführung des Verfahrens in einem bestimmten Bereich variiert werden. Man arbeitet im allgemeinen bei Temperaturen zwischen 0°C und 80°C, vorzugsweise zwischen 10°C und 60°C.

Bei der Durchführung des erfindungsgemäßen Verfahrens geht man im Allgemeinen so vor, dass man die veretherten Laktatester der Formel (I) mit einem oder mehreren Wirkstoffen sowie gegebenenfalls mit Zusatzstoffen vermischt. Die Reihenfolge, in der die Komponenten miteinander vermischt werden, ist beliebig.

Zur Durchführung des erfindungsgemäßen Verfahrens kommen übliche Geräte in Betracht, die zur Herstellung von agrochemischen Formulierungen eingesetzt werden.

Als Applikationsformen können alle, dem Fachmann als gebräuchlich bekannten Verfahren verwendet werden; beispielsweise genannt seien: Spritzen, Tauchen, Nebeln sowie eine Reihe spezieller Verfahren zur direkten unter- oder oberirdischen Behandlung von gesamten Pflanzen oder Teilen (Saatgut, Wurzel, Stolonen, Stängel, Stamm, Blatt), wie beispielsweise Stamminjektion bei Bäumen oder Stängelbandagen bei perennierenden Pflanzen, und eine Reihe spezieller indirekter Applikationsverfahren.

Der Begriff "Schadorganismen" umfasst alle Formen von Organismen, die im entsprechenden Einsatzgebiet ökonomische und/oder gesundheitliche Schäden verursachen. Bevorzugt sind pflanzliche und tierische Schadorganismen sowie Organismen, die Krankheiten verursachen, besonders bevorzugt sind terrestrische und aquatische Ungräser und Unkräuter, Algen, Moose, Insekten, Milben Nematoden, Nager, Pilze, Bakterien und Viren.

Die jeweilige flächen- und/oder objektbezogene Aufwandmenge der Pflanzenschutzmittel unterschiedlichster Formulierungstypen zur Bekämpfung der genannten Schadorganismen variiert sehr stark. Im Allgemeinen werden hierfür die, dem Fachmann als gebräuchlich für das jeweilige Einsatzgebiet bekannten Applikationsmedien in den gebräuchlichen Mengen eingesetzt; wie beispielsweise von mehreren hundert Liter Wasser pro Hektar bei Standard-Spritzverfahren über wenige Liter Öl pro Hektar bei der 'Ultra Low Volume'-Flugzeugapplikation bis hin zu wenigen Millilitern einer physiologischen Lösungen bei Injektionsverfahren. Die Konzentrationen der erfindungsgemäßen Pflanzenschutzmittel in den entsprechenden Applikationsmedien variieren daher in einem weiten Bereich und sind vom jeweiligen Einsatzgebiet abhängig. Im Allgemeinen werden Konzentrationen verwendet, die dem Fachmann als gebräuchlich für das jeweilige Einsatzgebiet bekannt sind. Bevorzugt sind Konzentrationen von 0,01 Gew.% bis 99 Gew.%, besonders bevorzugt von 0,1 Gew.% bis 90 Gew.%.

Die erfindungsgemäßen Pflanzenschutzmittel können z.B. in den für Flüssigpräparate üblichen Zubereitungsformen entweder als solche oder nach vorherigem Verdünnen mit Wasser ausgebracht werden, also z.B. als Emulsionen, Suspensionen oder Lösungen. Die Anwendung erfolgt dabei nach üblichen Methoden, also z.B. durch Verspritzen, Gießen oder Injizieren.

Die Aufwandmenge an den erfindungsgemäßen Pflanzenschutzmittel kann innerhalb eines größeren Bereiches variiert werden. Sie richtet sich nach den jeweiligen agrochemischen Wirkstoffen und nach deren Gehalt in den Pflanzenschutzmitteln.

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Bevorzugt sind Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten, die aus Teilen der Bäume hergestellt werden.

Der Begriff Nutzpflanzen, wie hier verwendet, bezeichnet Kulturpflanzen, die als Pflanzen für die Gewinnung von Nahrungsmitteln, Futtermitteln, Treibstoffen oder für technische Zwecke eingesetzt werden.

Zu den Nutzpflanzen, die erfindungsgemäß behandelt werden können, zählen z.B. folgende Pflanzenarten: Turf, Reben, Getreide, beispielsweise Weizen, Gerste, Roggen, Hafer, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung stellt keine Limitierung dar.

Als besonders geeignete Zielkulturen für die Anwendung des erfindungsgemäßen Verfahrens sind folgende Pflanzen anzusehen: Bamwolle, Aubergine, Turf, Kernobst, Steinobst, Beerenobst, Mais, Weizen, Gerste, Gurke, Tabak, Reben, Reis, Getreide, Birne, Bohnen, Sojabohnen, Raps, Tomate, Paprika, Melonen, Kohl, Kartoffel und Apfel.

Die erfindungsgemäß behandelten Pflanzen sind, soweit es den Einsatz an Herbiziden betrifft, alle Arten von Unkräutern. Was den Schutz von Kulturpflanzen durch Applikation von beispielsweise Fungiziden und Insektiziden betrifft, ist die Anwendung in wirtschaftlich bedeutenden, beispielsweise auch transgenen Kulturen von Nutz- und Zierpflanzen, z.B. von Getreide, wie Weizen, Gerste, Roggen, Hafer, Hirse, Reis, Maniok und Mais, oder auch Kulturen von Zuckerrübe, Baumwolle, Soja, Raps, Kartoffel, Tomate, Erbse und anderen Gemüsesorten bevorzugt.

Die Erfindung wird durch die Beispiele näher erläutert.

### Beispiele

### Herstellung der erfindungsgemäßen veretherten Laktatester

### Eingesetzte Rohstoffe:

Katalysator für die Alkylenoxidaddition (DMC-Katalysator):
Doppelmetallcyanid-Katalysator, enthaltend Zinkhexacyanocobaltat, tert.-Butanol und Polypropylenglykol mit einem zahlenmittleren Molekulargewicht von 1000 g/mol; beschrieben in WO-A 01/80994, Beispiel 6.

2-Ethylhexyllaktat, bezogen von der Firma Galactic
Lauryllaktat (PURASOLV LL®), bezogen von der Firma PURAC
IRGANOX® 1076: Octadecyl-3-(3,5-di-tert.butyl-4-hydroxyphenyl)propionat. (BASF SE))

### Herstellung der erfindungsgemäßen veretherten Laktatester auf Basis Ethylhexyllaktat

### A) 2-Ethylhexyllaktat 2PO/2EO

In einem 2 1 Laborautoklaven wurden bei 100°C unter Stickstoffatmosphäre 160,0 g (0,792 mol) 2-Ethylhexyllaktat und 0,067 g DMC-Katalysator vorgelegt. Nach 5-maligem Stickstoff/Vakuum-Austausch zwischen 0,1 und 3,0 bar (absolut) wurde auf 130°C aufgeheizt. Bei dieser Temperatur wurden dann unter Rühren innerhalb von 10 min 91,88 g (1,584 mol) PO in den Reaktor dosiert, wobei der Druck im Reaktor von 0,21 bar (absolut) auf 0,54 bar (absolut) anstieg. Nach einer Nachreaktionszeit von 25 min wurde der Reaktordruck zunächst mit Stickstoff auf 2,15 bar (absolut) eingestellt und anschließend unter Rühren bei 130°C innerhalb von 10 min 69,68 g (1,584 mol) EO in den Reaktor dosiert, wobei der Druck von 2,15 bar (absolut) auf 2,37 bar (absolut) anstieg. Nach einer Nachreaktionszeit von 45 min wurden leichtflüchtige Anteile bei 90°C für 30 min im Vakuum ausgeheizt und der Reaktionsansatz dann auf Raumtemperatur abgekühlt. Das Produkt wurde abschließend mit 161 mg IRGANOX® 1076: versetzt.

In analoger Weise wurden die Produkte der Zusammensetzung "2-Ethylhexyllaktat 2PO/5EO" sowie "2-Ethylhexyllaktat 2PO/10EO" aus Tabelle 1 hergestellt.

### B) 2-Ethylhexyllaktat 2EO

In einem 2 1 Laborautoklaven wurden bei 100°C unter Stickstoffatmosphäre 160,0 g (0,792 mol) 2-Ethylhexyllaktat und 0,07 g DMC-Katalysator vorgelegt. Nach 5-maligem Stickstoff/Vakuum-Austausch zwischen 0,1 und 3,0 bar (absolut) wurde auf 130°C aufgeheizt und der Reaktordruck dann mit Stickstoff auf 2,19 bar (absolut) eingestellt. Anschließend wurden unter Rühren bei 130°C innerhalb von 30 min 69,68 g (1,584 mol) EO in den Reaktor dosiert, wobei der Druck im Reaktor von 2,19 bar (absolut) auf 2,61 bar (absolut) anstieg. Nach einer Nachreaktionszeit von 60 min wurden leichtflüchtige Anteile bei 90°C für 30 min im Vakuum ausgeheizt und der Reaktionsansatz dann auf Raumtemperatur abgekühlt. Das Produkt wurde abschließend mit 115 mg IRGANOX® 1076: versetzt.

In analoger Weise wurden die Produkte der Zusammensetzung "2-Ethylhexyllaktat 5EO", "2-Ethylhexyllaktat 10EO" sowie "2-Ethylhexyllaktat 15EO" aus Tabelle 1 hergestellt.

### Herstellung der erfindungsgemäßen veretherten Laktatester auf Basis Lauryllaktat

### C) Lauryllaktat 5EO/2PO

In einen 2 1 Laborautoklaven wurden unter Stickstoffatmosphäre 50 g PURASOLV LL® gegeben. Nach Zugabe von 2 mg 85%-iger Phosphorsäure wurde der Reaktorinhalt 20 min bei Raumtemperatur gerührt (200 U/min, Propellerrührer). Nach Zugabe von 12 mg DMC-Katalysator wurde der Inhalt des Autoklaven auf 130 °C erwärmt und 30 min bei dieser Temperatur und Rühren mit 800 U/min im Vakuum bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann, ebenfalls bei 130 °C und Rühren mit 800 U/min, insgesamt 39,4 g Ethylenoxid über einen Zeitraum von 58 min eindosiert. Nach einer Nachreaktionszeit von 17 min wurden 20,8 g Propylenoxid über einen Zeitraum von 30 min bei 130 °C und Rühren mit 800 U/min eindosiert. Nach einer Nachreaktionszeit von 22 min wurde das Produkt noch 30 min bei einem absoluten Druck von 1 mbar ausgeheizt und danach auf 80 °C abgekühlt. Der Autoklav wurde mit Stickstoff auf ca. 1 bar entspannt. Aus dem Autoklaven wurden 50 g Produkt abgelassen und mit 30 mg IRGANOX® 1076 versetzt.

### D) Lauryllaktat 5EO/5PO

Der im Autoklaven verbliebene Inhalt des Produkts aus dem Beispiel C) wurde unter Rühren (800 U/min) auf 130 °C erhitzt, danach wurden weitere 17,1 g Propylenoxid über einen Zeitraum von 34 min eindosiert. Nach einer Nachreaktionszeit von 30 min wurde das Produkt noch 30 min bei einem absoluten Druck von 1 mbar ausgeheizt. Danach wurde mit Stickstoff auf ca. 1 bar entspannt und auf 80 °C abgekühlt. Das Produkt wurde abgelassen und mit 47 mg IRGANOX® 1076 versetzt.

### E) Lauryllaktat 8EO/2PO

In einen 2 1 Laborautoklaven wurden unter Stickstoffatmosphäre 152,0 g PURASOLV LL® gegeben. Nach Zugabe von 11 mg 85%-iger Phosphorsäure wurde der Reaktorinhalt 20 min bei Raumtemperatur gerührt (200 U/min, Propellerrührer). Nach Zugabe von 56 mg DMC-Katalysator wurde der Inhalt des Autoklaven auf 130 °C erwärmt und 30 min bei dieser Temperatur und Rühren mit 800 U/min im Vakuum bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann, ebenfalls bei 130 °C und Rühren mit 800 U/min, insgesamt 191,7 g Ethylenoxid über einen Zeitraum von 4,03 h eindosiert. Nach einer Nachreaktionszeit von 10 min wurden 63,2 g Propylenoxid über einen Zeitraum von 2,0 h bei 130 °C und Rühren mit 800 U/min eindosiert. Nach einer Nachreaktionszeit von 30 min wurde das Produkt noch 30 min bei einem absoluten Druck von 1 mbar ausgeheizt und danach auf 80 °C abgekühlt. Der Autoklav wurde mit Stickstoff auf ca. 1 bar entspannt. Aus dem Autoklaven wurden 76,9 g Produkt abgelassen und mit 41 mg IRGANOX® 1076 versetzt.

### F) Lauryllaktat 8EO/5PO

Der im Autoklaven verbliebene Inhalt des Produkts aus Beispiel E) wurde unter Rühren (800 U/min) auf 130 °C erhitzt, danach wurden weitere 77,2 g Propylenoxid über einen Zeitraum von 1,02 h eindosiert. Nach einer Nachreaktionszeit von 26 min wurde das Produkt noch 30 min bei einem absoluten Druck von 1 mbar ausgeheizt. Danach wurde mit Stickstoff auf ca. 1 bar entspannt und auf 80 °C abgekühlt. Das Produkt wurde abgelassen und mit 204 mg IRGANOX® 1076 versetzt.

### Verwendung

Die veretherten Laktatester besitzen sehr gute Eigenschaften als Tenside. Tenside finden unter anderem im Pflanzenschutz Einsatz als Netz- und Haftmittel sowie als Emulgatoren. Die Eignung als Netzmittel wird zum Beispiel durch die Statische Oberflächenspannung charakterisiert, die Eignung als Haftmittel durch die dynamische Oberflächenspannung (s. Adamson AW 1990. Physical Chemistry of Surfaces. London, Wiley / Berger PD & Berger CH, 1993. Effect of Surfactant Type and Order of Addition on Droplet Size and Dynamic Interfacial Properties. Pesticide formulations and application systems, 13th Vol., Berger, PD, Debisetty, BN, Hall, FR, Eds., American Society for testing and Materials. / Knowles, DA 1998. Chemistry and Technology of Agrochemical Formulations, Kluwer Academic Publishers, Dordrecht).

### 1) Statische Oberflächenspannung in wässrigen Systemen

Der im Gleichgewicht erreichbare Wert der Oberflächenspannung wurde über die Pending Drop Methode mit einem Goniometer (DSA10 Goniometer, Krüss) bestimmt. Die Tabelle zeigt die Ergebnisse der Messungen der veretherten Laktatester bei 0.3 und 3 g/l bei Raumtemperatur (20°C) im Vergleich zu Literaturwerten für zwei häufig verwendete Tenside.

**Tabelle 1**

| | **Statische Oberflächenspannung (mN/m)** | |
|---|---|---|
| **Testsubstanz** | 0.3 g/l | 3 g/l |
| Lauryllaktat 5EO/2PO | 31.63 | 31.34 |
| Lauryllaktat 5EO/5PO | 31.65 | 30.14 |
| Lauryllaktat 8EO/2PO | 30.76 | 30.71 |
| Lauryllaktat 8EO/5PO | 31.38 | 31.51 |
| 2-Ethylhexyllaktat 2PO/ 2EO | 40.40 | 29.47 |
| 2-Ethylhexyllaktat 2PO/ 5EO | 43.35 | 28.48 |
| 2-Ethylhexyllaktat 2PO/ 10EO | 47.43 | 36.0 |
| 2-Ethylhexyllaktat 2EO | 45.34 | 28.19 |
| 2-Ethylhexyllaktat 5EO | 48.65 | 32.61 |
| 2-Ethylhexyllaktat 10EO | 52.99 | 35.18 |
| 2-Ethylhexyllaktat 15EO | 54.33 | 42.23 |
| **Vergleich (kommerziell)** | | |
| Frigate * (Talgamin-ethoxylate) | - | 39.6 |
| Tanemul HOT ** (Ethylhexyl-Alkoxylat) | 35.2 | 29.7 |

| | | |
|---|---|---|
| *ISK Biosciences, Diegem (Belgien), **Tanatex, Leverkusen (Deutschland) | | |

Dabei bedeutet beispielsweise Lauryllaktat 5EO/2PO, dass für diesen veretherten Laktatester in der Schreibweise der Formel (I) R für Lauryl steht und R1 für -(-AO)ₘ-R' steht, wobei AO für ein Gemisch aus Ethylenoxid (EO) - und Propylenoxid (PO)-Resten steht, wobei der zuerst stehende EO-Anteil an die Laktatester-Gruppe bindet und der danach stehende PO-Anteil an die EO-Reste bindet, m für 5 + 2 = 7 steht und R' für Wasserstoff steht.

### 2) Dynamische Oberflächenspannung (Grenzflächenaktivität)

Die dynamische Oberflächenspannung wurde über die Blasendruckmethode bestimmt (Tensiometer BP2100, Krüss). Bei einer für die Spritzapplikation von Agrochemikalien in wässriger Verdünnung relevanten Zeitspanne (dem sogenannten Oberflächenalter in der Blasendruckmethode) von 200 Millisekunden korreliert der Wert der dynamischen Oberflächenspannung in [mN/m] mit der Haftung auf schwer benetzbaren Pflanzen wie Gerste (Getreide). Ein Wert von 50 mN/m (bei 20-21°C) ergibt gegenüber Wasser (72.8 mN/m) eine Verbesserung der Haftung von "Null Haftung" auf etwa 50% (Baur P, Pontzen R 2007. Basic features of plant surface wettability and deposit formation and the impact of adjuvants. In: R E Gaskin ed. Proceeding of the 8th International Symposium on Adjuvants for Agrochemicals. Publisher: International Society for Agrochemical Adjuvants (ISAA), Columbus, Ohio, USA). Tabelle 2 zeigt, dass dieser Wert schon bei der niedrigen Testkonzentration in Wasser von vielen veretherten Laktatestern erreicht wird und von allen bei 3 g/l klar unterschritten wird. Damit sind die veretherten Laktatester hervorragend geeignet, die Anlagerung von Agrochemikalien auf Getreide (mit Mais, Reis, Hirse), Banane, Kohl/Raps, Soja und anderen schwer benetzbaren Kultur- und Schadpflanzen zu fördern. Die positiven Netz- und Hafteffekte gelten natürlich auch für andere Organismen und künstliche Oberflächen bzw. technische Anwendungen etwa zur Erreichung dünner Beschichtungen auf oder der Reinigung von Oberflächen.

**Tabelle 2**

| | **Dynamische Oberflächenspannung (mN/m)** | |
|---|---|---|
| **Testsubstanz** | 0.3 g/l | 3g/l |
| Lauryllaktat 5EO/2PO | 51.9 | 37.4 |
| Lauryllaktat 5EO/5PO | 52.7 | 36.5 |
| Lauryllaktat 8EO/2PO | 54.3 | 37.7 |
| Lauryllaktat 8EO/5PO | 50.9 | 36.2 |
| 2-Ethylhexyllaktat 2PO/ 2EO | 47.8 | 30.7 |
| 2-Ethylhexyllaktat 2PO/ 5EO | 50.7 | 29.6 |
| 2-Ethylhexyllaktat 2PO/ 10EO | 54.2 | 37.4 |
| 2-Ethylhexyllaktat 2EO | 50.9 | 26.8 |
| 2-Ethylhexyllaktat 5EO | 54.4 | 35.3 |
| 2-Ethylhexyllaktat 10EO | 56.6 | 46.6 |
| 2-Ethylhexyllaktat 15EO | 58.6 | 48.1 |
| **Vergleich (kommerziell)** | | |
| Frigate* (Talgamin-ethoxylate) | - | 50.9 |
| Tanemul HOT** (Ethylhexyl-Alkoxylat) | 44.3 | 31.5 |

| | | |
|---|---|---|
| *ISK Biosciences, Diegem (Belgien), **Tanatex, Leverkusen (Deutschland) | | |

### 3) Förderung der Penetration beispielhaft ausgewählter Wirkstoffe

Tenside können auch die Aufnahme von (Wirk-)Stoffen durch Membranen wie Haut, Folien oder die pflanzliche Kutikula fördern. Als sogenannte "Finite-dose" -Applikation ist für die einmalige Applikation oder Auftragung einer Lösung, Creme, Gel etc. auf eine Membran bekannt, dass die Wirkstoffaufnahme auch nach erfolgter Benetzung durch manche Zusatzstoffe wie Tenside beeinflussbar ist. Dieser Effekt ist unabhängig von der Tensidwirkung, oft stark konzentrationsabhängig und findet größtenteils nach Verflüchtigung von Wasser und eventuell anwesenden Lösungsmitteln statt als Folge der Wechselwirkung z.B. mit Wirkstoff, Membran und Umweltfaktoren. Für verschiedene Tenside wird nach Zusatz zu Wirkstoffzubereitungen beobachtet, dass die Penetration eines bestimmten Wirkstoffes durch manche Tenside enorm gefördert wird während andere völlig unwirksam sind (Cronfeld, P, Lader, K. Baur, P. (2001). Classification of Adjuvants and Adjuvant Blends by Effects on Cuticular Penetration, Pesticide Formulations and Application Systems: Twentieth Volume, ASTM STP 1400, A. K. Viets, R. S. Tann, J. C. Mueninghoff, Edsl, American Society for Testing and Materials, West Conshohocken, PA 2001).

Das von der Tensidwirkung unabhängige Potential die Blattaufnahme von agrochemischen Wirkstoffen zu fördern wurde in Membranpenetrations-Versuchen mit Blattkutikeln von Apfel bestimmt. Das Prinzip der Methode ist veröffentlicht (z.B. WO-A-2005/194844) und nur die Spezifika und methodische Abweichungen werden nachfolgend erklärt. Die Blattkutikeln wurden auf die beschriebene Weise von Apfelblättern von Feldbäumen einer kommerziellen Kernobstanlage in Kriftel westlich von Frankfurt im Jahr 2010 enzymatisch isoliert. Die zunächst an der Luft getrockneten Kutikeln wurden in Diffusionszellen aus Edelstahl eingebaut. Nach Applikation auf die ursprüngliche Blattoberseite und Verdunstung der Testflüssigkeit, d.h. der wässrigen Zubereitungen der Wirkstoffe ohne oder mit den veretherten Laktatester wurden die Diffusionszellen in thermostatisierte Blöcke überführt und mit wässriger Flüssigkeit befüllt. Als Wasser zum Ansetzen der wässrigen Testflüssigkeiten wurde lokales Leitungswasser (bekannter Zusammensetzung) verwendet. In regelmäßigen Abständen wurden Proben genommen und abhängig vom Testsystem entweder per HPLC oder Szintillationsmessung der penetrierte Wirkstoffanteil bestimmt. Im System mit radioaktiv markiertem Wirkstoff (Thiacloprid und Fluoxastrobin) war die wässrige Flüssigkeit eine Phospholipidsuspension und die gesamte Menge wurde ausgetauscht. Bei der HPLC-Variante (Tebuconazol SC430) wurde nur ein Aliquot entnommen. Während des Versuchs waren die Temperatur im System (Block, Diffusionszellen, Flüssigkeiten etc.) und die Luftfeuchtigkeit über dem Spritzbelag auf der Kutikula exakt bekannt und kontrolliert. In den Versuchen wurde jeweils die relative Luftfeuchte durchgehend konstant bei 60% gehalten, die Temperatur aber nach einem Tag um 10°C erhöht und zwar von 20°C auf 30°C bei Thiacloprid und Tebuconazole, und von 15°C auf 25°C bei Fluoxastrobin.. Je Variante (Wirkstoff x, veretherten Laktatester) wurden 7-8 Wiederholungen angesetzt.

Beispielhaft sind nachfolgend die Aufnahmeförderung durch die veretherten Laktatester bei Tankmix-Zusatz zu
1) einer Lösung des Insektizides Thiacloprid mit 0.5 g/l und 3 g/l verethertem Laktatester
2) zu einem Suspensionskonzentrat des Fungizides Tebuconazole mit 0.5 g/l und 2 g/l verethertem Laktatester
3) einer Lösung des Fungizides Fluoxatrobin mit 0.5 g/l und 2 g/l verethertem Laktatester jeweils im Vergleich zu den Systemen ohne Zusatz der veretherten Laktatester dargestellt.

**Tabelle 3**

| | **Mittlere Penetration von Thiacloprid* in % (n= 4-8)** | | | |
|---|---|---|---|---|
| **Veretherter Laktatester** | **0.5 g/l** | | **3 g/l** | |
| | 24h** | 48h*** | 24h** | 48h*** |
| Thiacloprid gelöst in Aceton/Wasser ohne veretherten Laktatester | <3 | <5 | <3 | <5 |
| | | | | |
| Lauryllaktat 5EO/2PO | 13.6 | 17.4 | 63.9 | 74.5 |
| Lauryllaktat 5EO/5PO | 18.6 | 24.0 | 67.4 | 76.3 |
| Lauryllaktat 8EO/2PO | 14.5 | 18.8 | 73.3 | 80.4 |
| Lauryllaktat 8EO/5PO | 20.5 | 27.3 | 72.0 | 82.9 |
| 2-Ethylhexyllaktat 2PO/ 2EO | 11.5 | 15.1 | 20.3 | 23.4 |
| 2-Ethylhexyllaktat 2PO/ 5EO | 10.2 | 12.1 | 43.2 | 49.2 |
| 2-Ethylhexyllaktat 2PO/ 10EO | 8.1 | 10.0 | 36.9 | 47.2 |
| 2-Ethylhexyllaktat 2EO | 5.6 | 6.5 | 35.5 | 43.0 |
| 2-Ethylhexyllaktat 5EO | 12.1 | 14.1 | 31.4 | 39.9 |
| 2-Ethylhexyllaktat 10EO | 11.0 | 12.4 | 39.6 | 46.2 |
| 2-Ethylhexyllaktat 15EO | 10.5 | 14.0 | 29.9 | 41.9 |

| | | | | |
|---|---|---|---|---|
| * 0.2 g/l Thiacloprid; **20°C/60% relative Luftfeuchte (rel.LF); ***30°C/60% rel. LF | | | | |

**Tabelle 4**

| | **Mittlere Penetration von Tebuconazole* in % (n= 4-8)** | | | |
|---|---|---|---|---|
| **Veretherter Laktatester** | **0.5 g/l** | | **2 g/l** | |
| | 24h** | 48h*** | 24h** | 48h*** |
| Tebuconazole SC 430 ohne veretherten Laktatester | 2.2 | 20.5 | 2.2 | 20.5 |
| | | | | |
| Lauryllaktat 5EO/2PO | 27.6 | 75.1 | 57.1 | 68.3 |
| Lauryllaktat 5EO/5PO | 36.9 | 36.9 | 59.0 | 74.0 |
| Lauryllaktat 8EO/2PO | 25.4 | 62.9 | 62.9 | 73.3 |
| Lauryllaktat 8EO/5PO | 45.6 | 75.9 | 41.0 | 56.9 |
| 2-Ethylhexyllaktat 2PO/ 2EO | 23.2 | 58.1 | 27.0 | 36.5 |
| 2-Ethylhexyllaktat 2PO/ 5EO | 31.7 | 74.8 | 53.9 | 71.1 |
| 2-Ethylhexyllaktat 2PO/ 10EO | - | - | 52.2 | 79.3 |
| 2-Ethylhexyllaktat 2EO | - | - | 42.1 | 42.1 |
| 2-Ethylhexyllaktat 5EO | - | - | 45.0 | 63.8 |
| 2-Ethylhexyllaktat 10EO | - | - | 42.3 | 78.1 |
| 2-Ethylhexyllaktat 15EO | - | - | 30.5 | 53.6 |

| | | | | |
|---|---|---|---|---|
| * 0.5 g/l Tebuconazole; **20°C/60% rel. LF; ***30°C/60% rel. LF | | | | |

**Tabelle 5**

| | **Mittlere Penetration von Fluoxastrobin* in % (n= 4-8)** | | | |
|---|---|---|---|---|
| **Veretherter Laktatester** | **0.5 g/l** | | **2 g/l** | |
| | 24h** | 48h*** | 24h** | 48h*** |
| Fluoxastrobin gelöst in Aceton/Wasser ohne veretherten Laktatester | 2.6 | 6.9 | 2.6 | 6.9 |
| Kommerzielle Fluoxastrobin EC Formulierung 3 g/l | 1.8 | 7.3 | 1.8 | 7.3 |
| Lauryllaktat 5EO/2PO | 3.3 | 7.5 | 12.7 | 25.6 |
| Lauryllaktat 5EO/5PO | 4.7 | 10.0 | 12.2 | 23.1 |
| Lauryllaktat 8EO/2PO | 4.0 | 7.8 | 11.5 | 20.4 |
| Lauryllaktat 8EO/5PO | 3.9 | 9.6 | 6.8 | 19.8 |
| 2-Ethylhexyllaktat 2PO/ 2EO | 12.1 | 18.6 | 16.1 | 26.0 |
| 2-Ethylhexyllaktat 2PO/ 5EO | 9.9 | 16.2 | 14.4 | 24.2 |
| 2-Ethylhexyllaktat 2PO/ 10EO | 8.9 | 17.5 | 13.1 | 22.1 |
| 2-Ethylhexyllaktat 2EO | 10.6 | 35.2 | 11.4 | 18.9 |
| 2-Ethylhexyllaktat 5EO | 8.7 | 13.9 | 13.0 | 21.4 |
| 2-Ethylhexyllaktat 10EO | 9.7 | 15.9 | 16.8 | 26.9 |
| 2-Ethylhexyllaktat 15EO | 9.5 | 14.1 | 13.4 | 25.8 |

| | | | | |
|---|---|---|---|---|
| * 0.3 g/l Fluoxastrobin; **15°C/60% rel. LF; ***25°C/60% rel. LF | | | | |

Die Tabellen 3 bis 5 zeigen die hervorragende Eignung der erfindungsgemäßen veretherten Laktatester konzentrationsabhängig die Blattpenetration für verschiedene Wirkstoffe, Wirkstoffzubereitungen (Lösungen bei Thiacloprid und Fluoxastrobin; Suspensionskonzentrat bei Tebuconazole) und Umweltfaktoren stark zu fördern. Die relative Unabhängigkeit von dem Grad der Ethoxylierung oder Alkoxylierung bzgl. des Vermögens die Penetration zu fördern und die gleichzeitig unterschiedliche Grenzflächenaktivität macht die veretherten Laktatester auch zu interessanten, in agrochemische Formulierungen einzubauenden Komponenten.

### 4) Schaumverhalten

Zu den wichtigen Eigenschaften grenzflächaktiver Substanzen gehört das Schaumverhalten insbesondere in wässrigen Systemen. Praktisch alle Tenside schäumen und die Dauer bis zum Zusammenbrechen des Schaumes muß bei kritischen (stark schäumenden) Tensiden durch Zugabe von Entschäumern zu Formulierung oder wässriger Anwendungszubereitung verkürzt werden. Das Schaumverhalten einiger der erfindungsgemäßen Substanzen wurde mit wässrigen Lösungen bei einer Konzentration von 3 g/l nach dem Schaumtest der CIPAC Methode MT47 charakterisiert. Die Werte in der Tabelle geben die prozentuelle Füllung mit Schaum in einem Zylinder über einen Zeitraum von 12 Minuten wieder. Ein Wert von 100 (%) bedeutet also maximaler Schaum und wird zum Beispiel über den gesamten Zeitraum von 12 Minuten mit Laurylethersulfaten (wie Genapol LRO) erhalten.

Die nachfolgende Tabelle zeigt am Beispiel der erfindungsgemäßen veretherten Ethylhexyllaktatester, dass das Schaumverhalten der Substanzen als sehr günstig einzustufen ist. Bei der z.B. für den Einsatz in agrochemischen wässrigen Spritzbrühen relativ hohen Konzentration von 3 g/l bricht der Schaum sehr schnell zusammen. Überraschend ist auch bei den erfindungsgemäßen ethoxylierten Ethylhexyllaktatestern, dass nur nur der Ethoxylierungsgrad 5EO ein anfangs stärkerers Schaumverhalten zeigt, während höhere und niedere Ethoxylierungsgrade kaum bis keinen Schaum produzierten. Vergleich ist das Ergebnis mit den erfindungsgemäßen alkoxylierten Ethylhexyllaktatestern (gemischt EO / PO), obwohl diese auch noch stärkere Tenside sind (s. Tabelle 1 und 2)

**Tabelle**

| | **Prozentuales Schaumvolumen*** | | | |
|---|---|---|---|---|
| **Veretherter Laktatester** | 10s | 60s | 3 min | 12min |
| | | | | |
| Ethylhexyllaktat 2PO/ 2EO | 2 | 2 | 0 | 0 |
| Ethylhexyllaktat 2PO/ 5EO | 25 | 10 | 10 | 10 |
| Ethylhexyllaktat 2PO/ 10EO | 50 | 10 | 0 | 0 |
| 2-Ethylhexyllaktat 2EO | 5 | 5 | 5 | 5 |
| 2-Ethylhexyllaktat 5EO | 50 | 40 | 20 | 10 |
| 2-Ethylhexyllaktat 10EO | 10 | 5 | 5 | 0 |
| 2-Ethylhexyllaktat 15EO | 0 | 0 | 0 | 0 |

| | | | | |
|---|---|---|---|---|
| * Schaumtest nach CIPAC MT47 mit CIPAC D Wasser (342 ppm); Konzentration von 3 g/l | | | | |

### 5) Pflanzenverträglichkeit

Die veretherten Laktatester (Laktatester-Ethoxylate) waren bei den Konzentrationen, wo die deutlich fördernden Wirkungen auf Benetzung und Aufnahme von Agrochemikalien festegestellt wurden sehr gut pflanzenverträglich.

## Patentansprüche

1. Veretherte Laktatester der Formel (I) 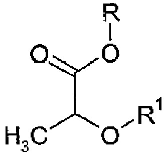 worin
R für 2-Ethyl-Hexyl oder Lauryl steht,
R¹ für einen alkoxylierten Alkylrest der Formel -(-AO)ₘ-R' steht, wobei AO für einen Ethylenoxid-Rest, einen Propylenoxid-Rest, einen Butylenoxid-Rest oder für Gemische aus Ethylenoxid und Propylenoxid-Resten steht und m für Zahlen von 2 bis 15 steht,
R' für Wasserstoff oder für einen verzweigten oder unverzweigten, gesättigten, teilweise gesättigten oder ungesättigten C₁-C₁₀-Alkylrest steht.

2. Veretherte Laktatester nach Anspruch 1, worin R für Lauryl steht, R¹ für -(-AO)ₘ-R' steht, wobei R' für Wasserstoff steht und -(-AO)ₘ aus der Gruppe bestehend aus den folgenden Alkyoxylat-Resten ausgewählt ist: -(EO)₅-(PO)₂, -(EO)₅-(PO)₅, - (EO)₈-(PO)₂, -(EO)₈-(PO)₅.

3. Veretherte Laktatester nach Anspruch 1, worin R für Ethylhexyl steht, R1 für -(-AO)ₘ-R' steht, wobei R' für Wasserstoff steht und -(-AO)ₘ aus der Gruppe bestehend aus den folgenden Alkyoxylat-Resten ausgewählt ist: -(EO)₂-(PO)₂,-(EO)₂-(PO)₅, -(EO)₂-(PO)₁₀, -(EO)₂, -(EO)₅, -(EO)₁₀, -(EO)₁₅.

4. Verfahren zur Herstellung der veretherten Laktatester nach einem der Ansprüche 1 bis 3, bei dem man Laktatester der Formel (II), in der R die in Anspruch 1 angegebenen Bedeutung hat und in der R2 für R' steht, wobei R' die in Anspruch 1 angegebene Bedeutung hat 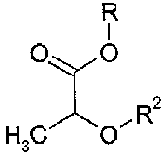 mit Alkylenoxiden in Gegenwart von DMC-Katalysatoren umsetzt.

5. Verwendung der veretherten Laktatester der Formel (I) nach Anspruch 1 als Tensid, als Netz und Haftmittel oder als Emulgator.

6. Verwendung von veretherten Laktatester der Formel (I) 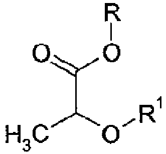 worin
R für unverzweigtes oder verzweigtes, gesättigtes oder ungesättigtes C₁-C₂₀-Alkyl steht, und
R¹ für einen alkoxylierten Alkylrest der Formel -(-AO)ₘ-R' steht, wobei AO für einen Ethylenoxid-Rest, einen Propylenoxid-Rest, einen Butylenoxid-Rest oder für Gemische aus Ethylenoxid und Propylenoxid-Resten oder für Gemische aus Ethylenoxidund Butylenoxid-Resten steht und
m für Zahlen von 2 bis 30 steht,
R' für Wasserstoff oder für einen verzweigten oder unverzweigten, gesättigten, teilweise gesättigten oder ungesättigten C₁-C₂₀ Alkylrest steht,
zur Verbesserung der Wirkung von agrochemischen Wirkstoffen in und auf Pflanzen.

7. Verwendung der veretherten Laktatester der Formel (I) nach Anspruch 1 zur Reduktion oder Vermeidung der Schaumbildung in agrochemischen Formulierungen.

## Claims

1. Etherified lactate esters of the formula (I) 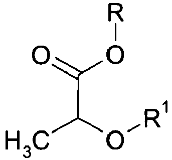 wherein
R is 2-ethylhexyl or lauryl,
R¹ is an alkoxylated alkyl radical of the formula -(-AO)ₘ-R', where
AO is an ethylene oxide radical, a propylene oxide radial, a butylene oxide radical, or mixtures of ethylene oxide and propylene oxide radicals, and m stands for numbers from 2 to 15,
R' is hydrogen or is a branched or unbranched, saturated, partly saturated or unsaturated C₁-C₁₀ alkyl radical.

2. The etherified lactate esters as claimed in claim 1, wherein R is lauryl, R¹ is -(-AO)ₘ-R', where R' is hydrogen and -(-AO)ₘ is selected from the group consisting of the following alkoxylate radicals: -(EO)₅-(PO)₂, -(EO)₅-(PO)₅, -(EO)₈-(PO)₂, -(EO)₈-(PO)₅.

3. The etherified lactate esters as claimed in claim 1, wherein R is ethylhexyl, R1 is -(-AO)ₘ-R', where R' is hydrogen and -(-AO)ₘ is selected from the group consisting of the following alkoxylate radicals: -(EO)₂-(PO)₂, -(EO)₂-(PO)₅, -(EO)₂-(PO)₁₀, -(EO)₂, -(EO)₅, -(EO)₁₀, -(EO)₁₅.

4. A process for preparing the etherified lactate esters as claimed in any of claims 1 to 3, which comprises reacting lactate esters of the formula (II) in which R has the definition given in claim 1 and in which R2 is R', where R' has the definition given in claim 1 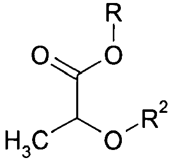 with alkylene oxides in the presence of DMC catalysts.

5. The use of the etherified lactate esters of the formula (I) as claimed in claim 1 as surfactant, as wetter and sticker, or as emulsifier.

6. The use of etherified lactate ester of the formula (I) 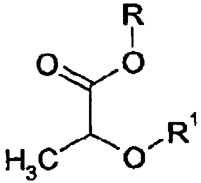 wherein
R is unbranched or branched, saturated or unsaturated C₁-C₂₀ alkyl, and
R¹ is an alkoxylated alkyl radical of the formula -(-AO)ₘ-R', where
AO is an ethylene oxide radical, a propylene oxide radial, a butylene oxide radical, or mixtures of ethylene oxide and propylene oxide radicals or mixtures of ethylene oxide and butylene oxide radicals, and
m stands for numbers from 2 to 30,
R' is hydrogen or is a branched or unbranched, saturated, partly saturated or unsaturated C₁-C₂₀ alkyl radical,
for improving the activity of active agrochemical ingredients in and on plants.

7. The use of the etherified lactate esters of the formula (I) as claimed in claim 1 for reducing or preventing foam formation in agrochemical formulations.

## Revendications

1. Esters lactate éthérifiés de formule (I) 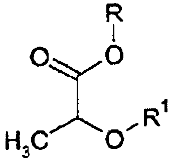 dans laquelle
R représente un groupe 2-éthyl-hexyle ou lauryle,
R¹ représente un radical alkyle alcoxylé de formule -(-AO)ₘ-R',
AO représentant un radical oxyde d'éthylène, un radical oxyde de propylène, un radical oxyde de butylène ou des mélanges de radicaux oxyde d'éthylène et oxyde de propylène et m représentant des nombres valant de 2 à 15,
R' représente un atome d'hydrogène ou un radical alkyle en C₁-C₁₀ ramifié ou non ramifié, saturé, partiellement saturé ou insaturé.

2. Esters lactate éthérifiés selon la revendication 1, dans lesquels R représente le groupe lauryle, R¹ représente un radical -(-AO)ₘ-R', R' représentant un atome d'hydrogène et -(-AO)ₘ étant choisi dans le groupe constitué par les radicaux alcoxylate suivants : -(EO)₅-(PO)₂, -(EO)₅-(PO)₅, -(EO)₈-(PO)₂, -(EO)₈-(PO)₅.

3. Esters lactate éthérifiés selon la revendication 1, dans lesquels R représente le groupe éthylhexyle, R¹ représente un radical -(-AO)ₘ-R', R' représentant un atome d'hydrogène et -(AO)ₘ étant choisi dans le groupe constitué par les radicaux alcoxylate suivants : -(EO)₂-(PO)₂, -(EO)₂-(PO)₅, -(EO)₂-(PO)₁₀, -(EO)₂, - (EO)₅, - (EO)₁₀, - (EO)₁₅.

4. Procédé pour la préparation des esters lactate éthérifiés selon l'une quelconque des revendications 1 à 3, dans lequel on fait réagir des esters lactate de formule (II), dans laquelle R a la signification indiquée dans la revendication 1 et dans laquelle R² représente R', R' ayant la signification indiquée dans la revendication 1 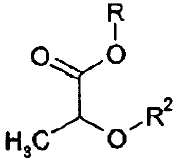 avec des oxydes d'alkylène en présence de catalyseurs DMC.

5. Utilisation des esters lactate éthérifiés de formule (I) selon la revendication 1, en tant que tensioactif, en tant qu'agent mouillant et adhésif ou en tant qu'émulsifiant.

6. Utilisation d'esters lactate éthérifiés de formule (I) 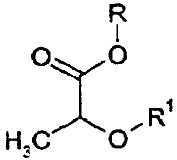 dans laquelle
R représente un groupe alkyle en C₁-C₂₀ ramifié ou non ramifié, saturé ou insaturé, et
R¹ représente un radical alkyle alcoxylé de formule -(-AO)ₘ-R', AO représentant un radical oxyde d'éthylène, un radical oxyde de propylène, un radical oxyde de butylène ou des mélanges de radicaux oxyde d'éthylène et oxyde de propylène ou des mélanges de radicaux oxyde d'éthylène et oxyde de butylène et
m représente des nombres valant de 2 à 30,
R' représente un atome d'hydrogène ou un radical alkyle en C₁-C₂₀ ramifié ou non ramifié, saturé, partiellement saturé ou insaturé,
pour l'amélioration de l'action de substances actives agrochimiques dans et sur des plantes.

7. Utilisation des esters lactate éthérifiés de formule (I) selon la revendication 1, pour réduire ou éviter la formation de mousse dans des compositions agrochimiques.
